# EUROPEAN PATENT SPECIFICATION

(11) **EP 2 773 275 B1**
(45) Date of publication and mention of the grant of the patent: **20.03.2019**
(21) Application number: 12783954.6
(22) Date of filing: 31.10.2012
(51) Int. Cl.: A61B 18/14, A61B 18/00, A61B 17/221

(54) **RF SURGICAL RESECTION INSTRUMENT HAVING A RESECTION LOOP FOR REMOVAL OF PATHOLOGICAL TISSUE**
CHIRURGISCHES RF -RESEKTIONSINSTRUMENT MIT EINER RESEKTIONSSCHLAUFE ZUR ENTNAHME VON PATHOLOGISCHEM GEWEBE
INSTRUMENT DE RÉSECTION CHIRURGICALE RADIOFRÉQUENCE (RF) AYANT UNE BOUCLE DE RÉSECTION POUR L'ENLÈVEMENT D'UN TISSU PATHOLOGIQUE

(30) Priority: 03.11.2011 DE 102011085721
(43) Date of publication of application: 10.09.2014
(73) Proprietor: Endox Feinwerktechnik GmbH, 72574 Bad Urach (DE); Farin, Günter, 72070 Tübingen (DE)
(72) Inventor: FARIN, Günter, 72070 Tübingen (DE); HERNIK, Matthias, 72574 Bad Urach (DE)
(74) Representative: Lohr, Jöstingmeier & Partner
(86) International application number: PCT/EP2012/071629
(87) International publication number: WO 2013/064577

(56) References cited:
- WO-A2-02/38052
- WO-A2-2011/012616
- US-A- 6 071 274

## Description

### Field of the invention

The invention relates to RF surgical resection instruments having asymmetrical resection loops such as are used, for example, for the endoscopically controlled resection of polyps or flat lesions of the mucosa of the gastro-intestinal tract.

### Description of the related art

An important requirement for endoscopically controlled resection, in particular of large (>2 cm) polyps or large (>2 cm) flat lesions of the mucosa of the gastro-intestinal tract, is the adherence to relevant requirements here on the part of oncology, namely, resection in sano, i.e. as far as into healthy tissue, and pathology, namely resection as far as possible en-bloc, i.e. in one piece, and below the first third of the submucosa (sm1) or as close as possible to the muscularis propria.

With the RF surgical resection loops corresponding to the prior art, so-called polypectomy snares, these requirements of oncology and pathology can only be satisfied with difficulty or not satisfied at all in cases of large polyps (>2 cm) or lesions (>2 cm) of the mucosa and, in particular, not when, prior to the RF surgical resection, electrically conductive liquids such as, for example, physiological NaCl solution is injected into the submucosa under these polyps or lesions in order to distance these polyps or lesions from the muscularis propria located thereunder, which must not be thermally damaged during the RF surgical resection. This problem results from the physics of endoscopic polypectomy (EPE).

Conventional polypectomy loops require an RF current of about 0.5 Aeff for a low-delay incision. RF generators of available RF surgical instruments can generate a maximum of 2 Aeff. Polyps or lesions having a circumference of about 4 cm or a diameter of about 1.3 cm can be resected herewith en-bloc with negligible incision delay. In cases of larger polyps or lesions, longer incision delays must be risked with the risk of thermal damage to the muscularis propria and perforation of the organ wall thereby caused. For this reason, in practice, polyps or lesions inter alia are only resected en-bloc up to a maximum diameter of 2 cm, where a still-acceptable incision delay is risked.

Polypos or lesions larger than 2 cm in diameter are either removed with polypectomy loops in several pieces (piece meal) or by the method of endoscopic submucosa dissection (ESD) en-bloc. Since piecemeal resection does not meet the requirements of pathology and only satisfies the requirements of oncology to a limited extent (high recurrence rate), and because ESD is technically very demanding and also very time-consuming and consequently expensive, alternative resection methods and suitable resection instruments for this are being sought.

WO 2011/012616 A2 discloses, inter alia, an asymmetric monopolar snare with which tissue to be removed can be ensnared and separated by RF surgery, comprising a short electrically non-insulated snare section which can be applied as RF surgical active electrode and a long electrically insulated snare section which cannot be applied as RF surgical active electrode, where the short and the long snare section are interconnected at their distal ends and where the long snare section is connected to a manipulation wire at its proximal end, with which this snare section can be drawn into a catheter and pushed out from this again, and the short snare section is connected to a stop wire at its proximal end so that the short snare section can only be pulled out from the distal end of the catheter as far as a stop, whilst the long snare section can be pushed further out from the distal end of the catheter, whereby the two snare sections form an opened snare.

This asymmetric monopolar snare is beset with two mutually contradictory problems. On the one hand, the short snare section should be as short as possible so that the RF current required for the RF surgical cutting, in particular during the incision phase and/or when an electrically conductive liquid, for example physiological NaCl solution is injected submucosally under the mucosa to be resected, is as far as possible smaller than the maximum RF current which can be generated by available RF generators, and on the other hand, the short snare section should be as long as possible so that the snare opens with the smallest possible shear when pushing out the long snare section.

In addition, in special cases of application it can be problematical to press this snare sufficiently firmly and flat and in a controlled fashion against the organ wall during the ensnarement and/or during the resection.

In addition, if this snare is not already ensnared around tissue, it can cut in an unintended direction.

WO 02/38052 A2 discloses steerable loop structures for supporting diagnostic and therapeutic elements in contact with body tissue.

### Summary of the invention

The invention is defined in the appended claims. Aspects, embodiments and examples disclosed herein which do not fall within the scope of the appended claims do not form part of the invention, and are merely provided for illustrative purposes.

It is the object of the invention to provide an RF surgical resection instrument comprising a loop as well as a resection loop, which is not beset with the problems listed above.

This object is solved by a device according to claim 1. Advantageous embodiments of the invention are given in the subclaims.

According to the invention, an RF surgical resection instrument comprises
- an asymmetrically opening and closing resection loop comprising
   ∘ a short electrically non-insulated loop section (RF surgical cutting and/or coagulation electrode or active electrode) having a proximal and a distal end and
   ∘ a long electrically insulated loop section having a proximal and a distal end,
      ▪ wherein the two loop sections consist of the same or of different material and their distal ends are mechanically and/or distally connected to one another,
- a rigid or flexible catheter made of electrically non-conductive material having a proximal and a distal end,
- a manipulation wire having a proximal and a distal end, which can be advanced inside the catheter in the axial direction from proximal to distal and can be withdrawn from distal to proximal and its distal end is connected mechanically and/or electrically to the proximal end of the long loop section or which is formed as an extension of the proximal end of the long loop section, whereby the short loop section is withdrawn from the distal end of the catheter when advancing the long loop section,
- a stop device comprising
   ∘ a stop wire having a proximal and a distal end, wherein the distal end of the stop wire is connected to the proximal end of the short loop section,
   ∘ a stop element which is movable relative to the catheter on the proximal end of the stop wire and
   ∘ a stop element which is fixed relative to the catheter inside the catheter, on which the stop element impacts when withdrawing the short loop section,
- an electrical insulation at least on a distal section of the stop wire or on the entire stop wire,
   ∘ wherein a distal electrically insulated section of the stop wire projects from the distal end of the catheter when the stop wire impacts on the stop device.
- an entrainer element comprising
   ∘ an entrainer element on the manipulation wire and
   ∘ an entrainer element on the stop wire.

When the loop is completely opened, the proximal end of the long loop section must project sufficiently far into the distal end of the catheter or at least the distal end of the manipulation wire must be electrically insulated because otherwise hot electrical arcs here can also damage the distal end of the catheter.

The invention is based on comprehensive technical and scientific investigations of a plurality of RF surgical loops and resections carried out therewith. Some of the results of these investigations have already been implemented in the RF surgical resection loops disclosed in WO 2011/012616 A2. In order to be able to also resect large polyps and large flat lesions of the mucosa endoscopically using resection loops en-bloc and close to the muscularis propria, an asymmetric resection loop is proposed in which the long loop section is electrically insulated and the short loop section is not electrically insulated. When the asymmetric loops are opened, the electrically non-insulated loop section according to WO 2011/012616 A2 extends from the distal end of the catheter to the distal end of the loop or the point where it is connected to the electrically insulated long loop section. This electrically non-insulated loop section serves as RF surgically active electrode and is generally also called cutting wire. The shorter the cutting wire, the smaller is the RF current required for the RF surgical cutting and in particular the RF current required for the shortest possible incision delay.

However, the electrically non-insulated loop section in the resection snares disclosed in WO 2011/012616 A2 cannot be made as short as would be expedient in particular in resections of polyps and flat lesions below which electrically conductive liquid, for example, physiological NaCl solution has been injected submucosally prior to the resection. Since, on account of the aforesaid requirements from oncology and pathology, this underinjection is becoming more and more standard even for smaller polyps and smaller flat lesions, it is also becoming necessary to make the non-electrically insulated loop section or cutting wire as short as possible. However, this conflicts with the fact that the shear force required to open asymmetrical resection loops is all the greater, the shorter is this cutting wire. From a critical length or shortness of the cutting wire, the loop can only be opened with very high shear force or no longer opened at all.

This problem is solved according to the invention whereby when the asymmetrical resection loop is opened, a piece of the stop wire that is electrically insulated projects from the distal end of the catheter and thus together with the RF surgically optimally short cutting wire is part of the optimally short loop section with regard to shear force.

Since now during the RF surgical resection, not the cutting wire but the electrically insulated stop wire, touches the distal end of the catheter, the distal end of the catheter can neither by damaged by a hot cutting wire nor by electrical arcs which are unavoidable during RF surgical cutting.

### Description of Drawings

The invention is described hereinafter as an example without restricting the general inventive idea by means of exemplary embodiments by reference to the drawings.
- Figure 1: shows an exemplary embodiment of a resection instrument according to the invention.
- Figure 2: shows in detail an exemplary embodiment of the connection of the two loop sections at the proximal end of the resection loop.
- Figure 3: shows a spur at the distal end of the resection loop
- Figure 4: shows a mandrel at the distal end of the resection loop

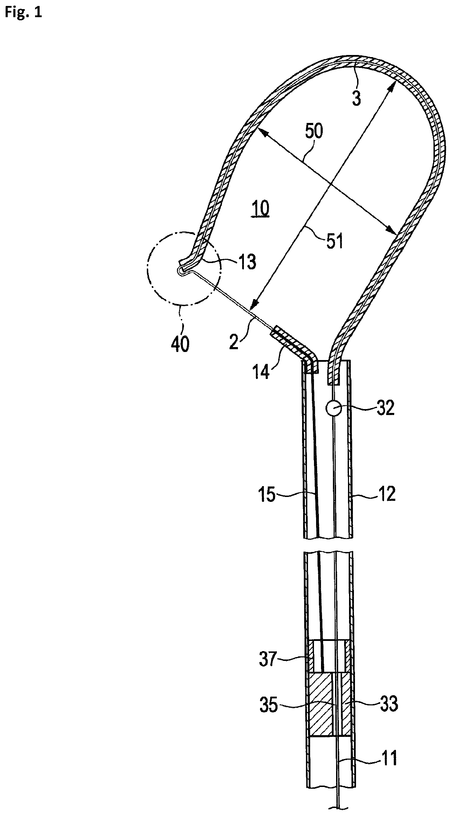

Figure 1 shows an exemplary embodiment of an RF surgical resection instrument according to the invention comprising an asymmetric resection loop 10, a catheter 12 and a stop device 33, 37, 15 as well as an entrainer device 32, 33, 15 and a manipulation wire 11 having a proximal and a distal end.

The asymmetrically opening and closing RF surgical resection loop 10 comprises a short, electrically non-insulated loop section 2 having a proximal end and a distal end as well as an electrically insulated long loop section 3 having a proximal and a distal end, which is surrounded by an electrical insulation 13. The long loop section 3 and the short loop section consist of the same or of different metal wires, preferably the long loop section 3 however consists of a spring-elastic metal wire and the short loop section 2 consists of flexible metal wire or flexible metal braid. The short loop section 2 and the long loop section 3 are mechanically and/or electrically interconnected at their distal ends.

The rigid or flexible catheter having a proximal and a distal end consists of electrically non-conductive material.

The manipulation wire consists of a metal wire which is preferably spring-elastic and torsionally rigid but can consist of the same material as the long loop section 3. The distal end of the manipulation wire 11 is connected to the proximal end of the long loop section 3.

The stop device comprises a stop 37 fixed in the catheter, a stop element 33 which is movable in the catheter and a stop wire 15 having a proximal and a distal end. The proximal end of the stop wire 15 is connected to the stop element 33 and the distal end of the stop wire is connected to the proximal end of the short loop section 2. The stop wire 15 and the short loop section 2 consist of the same or of different material.

The stop wire 15 is surrounded by an electrical insulation 14 at least at its proximal end section or over its entire length.

The stop device delimits the maximum length which the short, electrically non-insulated loop section 2 including a proximal electrically insulated end section 14 of the stop wire 15 during sliding of the long loop section out from the distal end of the catheter 12 in order to open the loop 10 by further sliding out the long loop section 3.

The shear force required to open an asymmetrical resection loop is greatest when the long loop section has pulled out the short loop section as far as its stop from the distal end of the catheter so that the section of the long loop section slid out from the distal end of the catheter is the same length as the short loop section. This phenomenon is not relevant in conventional asymmetric resection loops in which the long loop section is not electrically insulated and which have already been in use for more than 30 years, because in these resection loops, the long loop section is not electrically insulated and it therefore makes no sense in these loops to make the short loop section as short as possible in order to hereby make the RF current required for the RF surgical resection and in particular during the incision phase as small as possible.

In order to make the short non-electrically insulated loop section in the resection loop according to the invention as short as possible with regard to the lowest possible RF current required for the RF surgical resection, without making the shear force required for opening this resection loop too great, a sufficiently long proximal section of the stop wire 15 with the electrical insulation 14 is withdrawn from the distal end of the catheter until the entrainer or movable stop element 33 stops against the stop 37 fixed in the catheter.

In this way, it is also prevented that hot electrical arcs between the short electrically non-insulated loop section 2 and tissue, which are required to cut tissue, thermally damage the distal end of the catheter 12 and in particular that the wire of the short loop section heated by the hot electrical arcs melts into the distal end of the catheter, in particular as long as the short non-electrically insulated loop section is pressed onto the wall of the catheter when the resection loop is opened.

The entrainer device comprises an entrainer 32 fixed on the manipulation wire 12 and the stop element 33 required for the stop device already described above which is connected to the stop wire 15. This entrainer device draws the short loop section into the catheter as soon as the entrainer 32 impacts against the stop element 33 when withdrawing the manipulation wire 11 and therefore also the long loop section 3 into the catheter.

With the asymmetric resection loop according to the invention, theoretically arbitrarily large polyps of flat lesions of the mucosa of the gastrointestinal tract following submucosal underinjeciton can be resected en-bloc, i.e. in one piece, with low RF current and negligible incision delay near the muscularis propria, where this loop can be pressed against the organ wall during the RF surgical resection. Another advantage of this resection loop is that when used as intended, this only cuts parallel to the organ wall or to the muscularis propria and not in the direction of the organ wall, thus avoiding perforations of the organ wall.

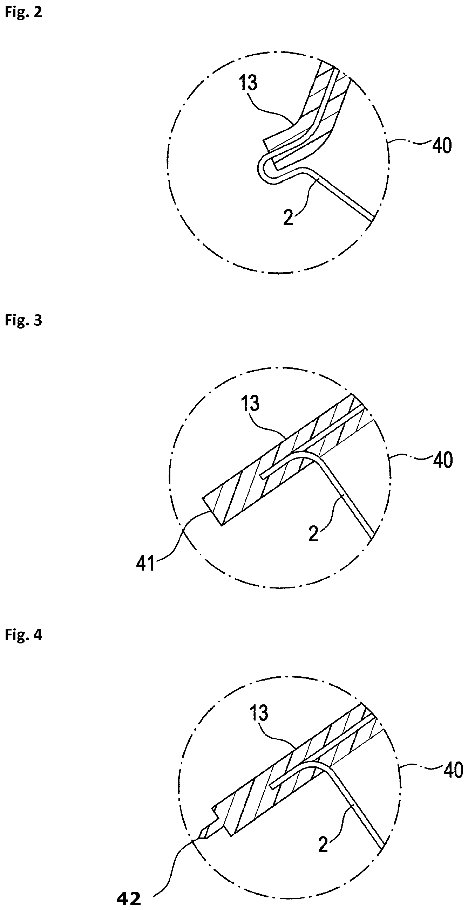

In order to prevent this loop cutting into the organ wall, for example, when not used as intended or accidentally, the resection loop according to the invention, as shown schematically in Fig. 3 can be fitted at its distal end 40 with an electrically non- conductive spur 41 which is so long that it prevents any contact of the short loop section, for example, with the organ wall as is possible with the distal end of the loop shown schematically in Fig. 2.

In order to be able to press the resection loop according to the invention in a manner as controlled as possible and sufficiently firmly against the organ wall around polyps or flat lesions when this is applied around polyps or flat lesions of the mucosa to be resected, this resection loop according to the invention, as shown schematically in Fig. 4, is fitted with an electrically non-conductive mandrel 42 at its distal end 40, which can be inserted into the mucosa quasi as a pivot point for the resection loop in the vicinity of a polyp or a flat lesion of the mucosa in order thereafter to tilt the opened loop about this pivot point above the polyp or the flat lesion. A torsionally rigid manipulation wire which has already been mentioned above can be used for this.

### List of reference numerals

- 2: Short electrically non-insulated loop section
- 3: Long electrically insulated loop section
- 10: Loop or resection loop
- 11: Manipulation wire
- 12: Catheter
- 13: Electrical insulation of the electrically long loop section 3
- 14: Electrical insulation on the stop wire 15
- 15: Stop wire
- 32: Entrainer
- 33: Stop element which is movable in the catheter
- 35: Hole
- 37: Fixed stop in the catheter
- 40: Distal end of the resection loop 10
- 41: Spur
- 42: Mandrel
- 50, 51: Opening width of the resection loop 10

## Claims

1. An RF surgical resection instrument comprising
- an asymmetric opening and closing resection loop (10) for ensnaring and RF surgical separation of tissue, further comprising a short, electrically non-insulated loop section (2) having a proximal and a distal end, and a long electrically insulated loop section (3) having a proximal and a distal end, wherein the distal ends of the loop sections are mechanically and/or electrically interconnected ,
- a catheter (12) made of electrically non-conductive material, having a proximal end and a distal end, and an opening in the longitudinal direction between the ends,
- a manipulation wire (11) in the catheter (12), having a proximal and a distal end, which can be advanced from proximal to distal inside the catheter in the longitudinal direction and can be withdrawn from distal to proximal, and its distal end is mechanically and/or electrically connected to the proximal end of the long loop section or which is formed as an extension of the proximal end of the long loop section, wherein the short loop section (2) is pulled out from the distal end of the catheter (12) during advance of the long loop section (3),
- a stop device comprising a stop wire (15) having a proximal and a distal end, wherein the distal end of the stop wire is connected to the proximal end of the short loop section (2), a first entrainer (33) at the proximal end of the stop wire (15) which is movable relative to the catheter (12), a stop (37) which is fixed relative to the catheter (12), inside the catheter, on which the entrainer (33) impacts when pulling out the short loop section (2) so that the stop wire can be pulled out from the distal end of the catheter with a predefined length, and,
- an entrainer device comprising the first entrainer (33) and a second entrainer (32) which is connected to the distal end of the manipulation wire (11), wherein upon withdrawal of the manipulation wire the second entrainer (32) withdraws the first entrainer (33) and therefore also draws the stop wire (15) into the catheter
**characterized in that**
the stop wire (15) has an electrical insulation (14) at least on the section which can be pulled out from the catheter or over its entire length so that the insulation projects from the distal end of the catheter, when the first entrainer (33) impacts on the stop (37).

2. The monopolar RF surgical resection loop (10) according to claim 1, **characterized in that** the distal end of the loop is formed as a spur.

3. The monopolar RF surgical resection loop (10) according to claim 1, **characterized in that** the distal end of the loop is formed as a mandrel.

4. RF surgical resection instrument comprising a monopolar RF surgical resection loop according to any one of the preceding claims and a handle at a proximal end of the catheter (12).

## Patentansprüche

1. Ein HF-chirurgisches Resektionsinstrument, umfassend
- eine asymmetrische öffnende und schließende Resektionsschlinge (10) zum Umschlingen und zum HF-chirurgischen Abtrennen von Gewebe, weiterhin umfassend einen kurzen, elektrisch nicht-isolierten Schlingenteil (2) mit einem proximalen und einem distalen Ende, und einem langen elektrisch isolierten Schlingenteil (3) mit einem proximalen und einem distalen Ende, wobei die distalen Enden der Schlingenteile mechanisch und/oder elektrisch miteinander verbunden sind,
- einen Katheter (12) aus elektrisch nicht-leitfähigem Material, mit einem proximalen und einem distalen Ende, und einer Öffnung in Längsrichtung zwischen den Enden,
- einen Manipulationsdraht (11) in dem Katheter (12), mit einem proximalen und einem distalen Ende, welcher innerhalb des Katheters in Längsrichtung von proximal zu distal vorwärtsbewegt werden kann und von distal zu proximal zurückgezogen werden kann, und dessen distales Ende mechanisch und/oder elektrisch mit dem proximalen Ende des langen Schlaufenabschnittes verbunden ist oder welcher als eine Verlängerung des proximalen Endes des langen Schlaufenabschnittes geformt ist, wobei der kurze Schlaufenabschnitt (2) aus dem distalen Ende des Katheters (12) während des Vorwärtsbewegens des langen Schlaufenabschnittes (3) herausgezogen wird,
- eine Anschlagvorrichtung umfassend einen Anschlagdraht (15) mit einem proximalen und einem distalen Ende, wobei das distale Ende des Anschlagdrahtes mit dem proximalen Ende des kurzen Schlingenteils (2) verbunden ist, einem ersten Mitnehmer (33) an dem proximalen Ende des Anschlagdrahtes (15), welcher relativ zu dem Katheter (12) beweglich ist, einem Anschlag (37), der relativ zu dem Katheter (12) fixiert ist, innerhalb des Katheters, auf welchen der Mitnehmer (33) einwirkt, wenn der kurze Schlaufenabschnitt (2) herausgezogen wird, so dass der Anschlagdraht aus dem distalen Ende des Katheters mit einer vordefinierten Länge herausgezogen werden kann, und,
- eine Mitnehmervorrichtung umfassend den ersten Mitnehmer (33) und einen zweiten Mitnehmer (32), welcher mit dem distalen Ende des Manipulationsdrahtes (11) verbunden ist, wobei beim Zurückziehens des Manipulationsdrahtes der zweite Mitnehmer (32) den ersten Mitnehmer (33) zurückzieht und damit ebenso den Anschlagdraht (15) in den Katheter hinein zieht.
**dadurch gekennzeichnet, dass**
der Anschlagdraht (15) eine elektrische Isolierung (14) zumindest auf dem Abschnitt hat, der aus dem Katheter herausgezogen werden kann, oder über seine gesamte Länge, so dass die Isolierung vom distalen Ende des Katheters hervorsteht, wenn der erste Mitnehmer (33) auf den Anschlag (37) stößt.

2. Die monopolare HF-chirurgische Resektionsschlinge (10) nach Anspruch 1,
**dadurch gekennzeichnet, dass**
das distale Ende der Schlinge als ein Sporn ausgebildet ist.

3. Die monopolare HF-chirurgische Resektionsschlinge (10) nach Anspruch 1,
**dadurch gekennzeichnet, dass**
das distale Ende der Schlinge als ein Dorn ausgebildet ist.

4. HF-chirurgisches Resektionsinstrument, umfassend eine monopolare HF-chirurgische Resektionsschlinge nach einem der vorhergehenden Ansprüche und einen Griff an einem proximalen Ende des Katheters (12).

## Revendications

1. Instrument de résection chirurgicale RF comprenant
- une boucle de résection d'ouverture et de fermeture asymétrique (10) pour la capture et la séparation chirurgicale RF de tissu, comprenant en outre une section de boucle courte non isolée électriquement (2) ayant une extrémité proximale et une extrémité distale, et une section de boucle longue isolée électriquement (3) ayant une extrémité proximale et une extrémité distale, dans lequel les extrémités distales des sections de boucle sont interconnectées mécaniquement et/ou électriquement,
- un cathéter (12) en matériau non conducteur électriquement, ayant une extrémité proximale et une extrémité distale, et une ouverture dans la direction longitudinale entre les extrémités,
- un fil de manipulation (11) dans le cathéter (12), ayant une extrémité proximale et distale, qui peut être avancé de l'extrémité proximale à l'extrémité distale à l'intérieur du cathéter dans la direction longitudinale et peut être retiré de l'extrémité distale à l'extrémité proximale, et son extrémité distale est connectée mécaniquement et/ou électriquement à l'extrémité proximale de la section de boucle longue ou qui est formée comme un prolongement de l'extrémité proximale de la section de boucle longue, dans lequel la section de boucle courte (2) est sortie de l'extrémité distale du cathéter (12) pendant l'avancée de la section de boucle longue (3),
- un dispositif de butée comprenant un fil de butée (15) ayant une extrémité proximale et une extrémité distale, dans lequel l'extrémité distale du fil de butée est connectée à l'extrémité proximale de la section de boucle courte (2), un premier entraînement (33) au niveau de l'extrémité proximale du fil de butée (15) qui est mobile par rapport au cathéter (12), une butée (37) qui est fixée par rapport au cathéter (12), à l'intérieur du cathéter, sur laquelle l'entraînement (33) a un impact lors de la sortie de la section de boucle courte (2) de sorte que le fil de butée puisse être sorti de l'extrémité distale du cathéter avec une longueur prédéfinie, et,
- un dispositif d'entraînement comprenant le premier entraînement (33) et un second entraînement (32) qui est connecté à l'extrémité distale du fil de manipulation (11), dans lequel lors du retrait du fil de manipulation, le second entraînement (32) retire le premier entraînement (33) et par conséquent attire également le fil de butée (15) dans le cathéter
**caractérisé en ce que**
le fil de butée (15) a une isolation électrique (14) au moins sur la section qui peut être sortie du cathéter et sur sa longueur entière de sorte que l'isolation dépasse de l'extrémité distale du cathéter, lorsque le premier entraînement (33) a un impact sur la butée (37).

2. Boucle de résection chirurgicale RF monopolaire (10) selon la revendication 1, **caractérisée en ce que** l'extrémité distale de la boucle est formée comme un éperon.

3. Boucle de résection chirurgicale RF monopolaire (10) selon la revendication 1, **caractérisée en ce que** l'extrémité distale de la boucle est formée comme un mandrin.

4. Instrument de résection chirurgicale RF comprenant une boucle de résection chirurgicale RF monopolaire selon l'une quelconque des revendications précédentes et un manche au niveau d'une extrémité proximale du cathéter (12).
